# EUROPÄISCHE PATENTANMELDUNG

(11) **EP 0 919 717 A1**
(43) Veröffentlichungstag der Anmeldung: **02.06.1999**
(21) Anmeldenummer: 98120882.0
(22) Anmeldetag: 04.11.1998
(51) Int. Cl.: F03G 7/06, A61B 17/72

(54) **Antriebsvorrichtung mit einem aus einer Formgedächtnislegierung geformten Element sowie deren Verwendung**

(30) Priorität: 27.11.1997 DE 19752560; 12.03.1998 DE 19810639
(71) Anmelder: Fachhochschule Konstanz, 78462 Konstanz (DE)
(72) Erfinder: Gümpel, Paul Prof.Dr.-Ing., 78351 Bodman-Ludwigshafen (DE); Kühn, Harald Dipl.-Phys., 8280 Kreuzlingen (CH); Strittmatter, Joachim Dipl.-Ing. (FH), 78464 Konstanz (DE); Hütterer, Heike Dipl.-Ing., 78467 Konstanz (DE)
(74) Vertreter: Hiebsch, Gerhard F., Dipl.-Ing.

(57) **Zusammenfassung**

Bei einer Antriebsvorrichtung mit einer ein aus einer Formgedächtnislegierung (FGL) geformtes Element (24) und einen letzterem zugeordneten, einer Arbeitsbewegung entgegenwirkenden Kraftspeicher (30) aufweisenden Antriebseinheit ist das Element (24) unter Temperatureinwirkung gegen den Kraftspeicher (30) längenveränderlich ausgebildet sowie endwärts jeweils einem zu ihm querschnittsverschiedenen Tragorgan (22,22ₑ) zugeordnet; der Kraftspeicher (30) ist zwischen den Tragorganen (22,22ₑ) spannbar, wobei zumindest eines der Tragorgane mit Greifelementen (32) versehen ist, die mit Gegenelementen (34) eines zu den Tragorganen (22,22ₑ) relativ bewegbaren Widerlagers (12) kämmend zusammenwirken. Die Greifelemente (32) der Tragorgane (22,22ₑ) sowie die Gegenelemente (34) des Widerlagers (12) bilden als Eingriffspartner einen auf Reibungsanisotropie beruhenden Raupenmechanismus zur Erzeugung eines definierten Hubes zwischen Tragorgan (22,22ₑ) und Widerlager (12) bei Durchlaufen eines Temperaturzyklus der FGL.

## Beschreibung

Die Erfindung betrifft eine Antriebsvorrichtung mit einer Antriebseinheit, die ein aus einer Formgedächtnislegierung -- mit Einweg- oder Zweiwegeffekt -- geformtes Element und einen letzterem zugeordneten, einer Arbeitsbewegung entgegenwirkenden Kraftspeicher enthält, wobei das Element unter Temperatureinwirkung gegen den Kraftspeicher längenveränderlich ausgebildet ist. Zudem erfaßt die Erfindung Verwendungen für diese Antriebsvorrichtung.

Mit sog. Formgedächtnis- (Shape-Memory) Legierungen ist die Möglichkeit gegeben, Wärmeenergie direkt in mechanische Energie umzuwandeln. Wird eine Formgedächtnislegierung (FGL) bei Temperaturen unterhalb einer bestimmten kritischen Temperatur bleibend verformt, so kann sie sich bei der Erwärmung über diese kritische Temperatur an ihre ursprüngliche Gestalt erinnern und diese wieder annehmen.

Allgemein bekannt ist die Verwendung von Drähten und/oder Federn aus Formgedächtnislegierungen mit Zweiweg-Effekt, die abwechselnd in kaltes und warmes Wasser tauchen. Durch die Längenänderung der Drähte wird ein rotierender Antrieb in Bewegung gesetzt. Derartige Antriebseinheiten -- deren Einsatz mit beispielsweise bis 10⁸ Umwandlungszyklen in Dauerprüfung gestestet worden ist -- können eine mechanische Energie um 1000 W abgeben.

Linearantriebe mit Elementen aus einer Formgedächtnislegierung zeichnen sich oft durch einen auf wenige Millimeter begrenzten Arbeitsweg und durch eine geringe Leistung aus. Zudem ist bei bekannten Linearantrieben mit elektrischem Antrieb beispielsweise eine Umsetzung von Rotationsbewegung in eine lineare Bewegung notwendig. Dies verursacht einen relativ komplexen mechanischen Aufbau und die Notwendigkeit des Einsatzes von Schmierstoff. Weiterhin treten fertigungstechnische Schwierigkeiten bei der Miniaturisierung auf.

Die Schrift zu DE-U-91 00 339 schildert einen derartigen Linearantrieb mit einer in Längsrichtung beweglich an einem Rahmen geführten und beidseitig über diesen hinausragenden Betätigungsstange, an der ein mit einem Heizelement ausgestattetes Federelement aus einer Formgedächtnislegierung sowie eine deren Arbeitsbewegung entgegenwirkende Rückstellfeder angreifen. Die Rückstellfeder sowie das Federelement stützen sich über Anlageflächen am Rahmen ab. Dieses Federelement -- bevorzugt aus 48,8 Gew.-% Ni, 45,2 Gew.-% Ti und 6,0 Gew.-% Cu -- ist auf einem Teil seiner Längserstreckung von einem elektrisch leitenden sowie gut wärmeleitenden metallischen Rohrelement mit Vierkant-Außenprofil umschlossen, an dessen Außenseite als PCT-Bauteil ein plattenförmiges Heizelement anliegt. Rohrelement und Heizelement stehen jeweils über eine an ihnen in Anlage gehaltene Federzunge eines Elektrokontaktes mit diesem in Verbindung.

Nach der DE-A-37 31 146 wird bei einer -- vor allem als Schalterantrieb oder allgemein als Antriebsmechanismus einsetzbaren -- Antriebseinrichtung für reversible Bewegungen ein zweiteiliges Element aus einer Formgedächtnislegierung wirksam, dessen Teilelemente bezüglich ihrer Arbeitsrichtung gegensinnig ausgewählt und angeordnet sind. Die Teilelemente bestehen aus zumindest gleichartigem Material mit Einwegeffekt in einer Gestalt für große Wegänderung und sind endwärts bewegungsbezogen festgelegt sowie durch ein als Antriebskörper nutzbares Koppelglied verbunden. Sie werden zeitlich nacheinander auf ihre Arbeitstemperatur gebracht.

In Kenntnis dieses Standes der Technik hat sich der Erfinder die Aufgabe gestellt, einen kostengünstigen, mechanisch einfach aufgebauten, leicht zu miniaturisierenden Antrieb - - insbesondere einen Linearantrieb -- mit großem Arbeitsweg, variabler Positionierung und hoher Leistung zu schaffen.

Zur Lösung dieser Aufgabe führt die Lehre der unabhängigen Patentansprüche; die Unteransprüche geben günstige Weiterbildungen an.

Erfindungsgemäß ist das aus der Formgedächtnislegierung geformte Element endwärts jeweils einem zu ihm querschnittsverschiedenen Tragorgan zugeordnet und der Kraftspeicher zwischen den Tragorganen spannbar, wobei zumindest eines der Tragorgane mit einem zu ihm relativ bewegbaren Widerlager geführt ist sowie bei Kraft- und/oder Formschluß zusammenwirkt; bevorzugt soll zumindest eines der Tragorgane mit Greifelementen versehen sein, die mit Gegenelementen jenes zu den Tragorganen relativ bewegbaren Widerlagers kämmend zusammenwirken. Solche Widerlager können -- wie weiter unten erörtert -- die Antriebseinheit umfangende Hohlprofile oder sie durchsetzende Körper --beispielsweise Kolben -- sein, jedoch ist es auch möglich, die Widerlager als der Antriebseinrichtung von außen her zugeordnete Walzen, Scheiben od.dgl. bewegte Körper bzw. Flächenelemente auszubilden.

Vorteilhafterweise bilden bei einer Ausgestaltung die Greifelemente der Tragorgane sowie die Gegenelemente des Widerlagers als Eingriffspartner einen auf Kraftschluß beruhenden Raupenmechanismus oder aber -- nach einer anderen Ausführung der Erfindung -- einen auf Reibungsanisotropie beruhenden Raupenmechanismus, zur Erzeugung eines definierten Hubes zwischen Tragorgan und Widerlager bei Durchlaufen eines Temperaturzyklus des Elementes aus der Formgedächtnislegierung.

Als günstig hat sich eine die Reibungsanisotropie vermittelnde formschlüssige Rutschverzahnung aus Fasern, ausgerichteten Lamellen oder Stufen als Eingriffspartner erwiesen. Bevorzugt aber werden als letztere Sägezahnrillen mit ihnen zugeordneten Sperrklinken.

Durch die Verwendung eines Raupenmechanismus wird ein großer Arbeitsweg -- bei gleichzeitiger Reduktion der beweglichen Teile auf Kolben und Antriebseinheit -- erreicht. Dabei kann sich die Antriebseinheit -- erfindungsgemäß mit in Form eines Hohlprofils aus Formgedächtnislegierung hergestelltem Element -- im Inneren eines reibungsanisotropen Hohlkörpers oder Hohlstranges bewegen, dessen Länge den Arbeitsweg begrenzt; der rohrartige, in einer Längsachse verschieblich zwischen zwei quer zur Längsachse verlaufenden scheibenartigen Tragorganen gelagerte Hohlstrang weist innenseitig einen der Eingriffspartner auf, während der andere am Tragorgan zu finden ist.

Bei einer weiteren Ausführung kann eines der Tragorgane mit einem axial beweglich gelagerten strangartigen Kolben verbunden, bei einer anderen der Kolben mit einer Sackbohrung zur Aufnahme eines Abschnittes des Hohlprofiles ausgestattet sein.

Aus Gründen der Sicherheit und Stabilität soll der Kolben zudem in wenigstens einem Gleitring geführt werden, der bevorzugt im Hohlstrang oder Rohr festliegt. Der gleichen Sicherheit dient ein weiteres Merkmal, wonach der Kolben an seinem Umfang mit zumindest einer Spiralnut versehen ist, die mit wenigstens einer vom Hohlstrang einwärts ragenden Nase kämmt.

Soll die lineare Bewegung des Kolbens mit einer Rotation überlagert werden, weist der Kolben wenigstens eine achsparallele Kolbennut für eine Führungsnase des Hohlstranges auf; die Nase/n ragt/ragen vorteilhafterweise radial vom Gleitring ab.

Es liegt auch im Rahmen der Erfindung, daß das Hohlprofil ein von einer Schraubenfeder als Kraftspeicher umfangener Hohlzylinder und/oder der Hohlstrang ein Rohr sind/ist. Dazu hat es sich als günstig erwiesen, im Hohlraum des Hohlzylinders wenigstens ein Heizelement zu lagern.

Bei einer anderen erfindungsgemäßen Ausgestaltung ist die Antriebseinheit ebenfalls ein Hohlprofil bzw. Hohlzylinder aus Formgedächtnislegierung, jedoch wird ihr zentrischer Hohlraum von einem Kolben axial durchsetzt, wobei der Kolben einen Eingriffspartner aufweist, der mit dem anderen Eingriffspartner im Ringdurchbruch wenigstens einer Ringscheibe als Tragorgan kämmt. Hier liegt die Wegstrecke des Raupensystems im Inneren des FGL-Elementes. Diese Vorgabe berücksichtigt u.a., daß hohe Kräfte durch Verwendung großer Querschnitte der Elemente aus der Formgedächtnislegierung erzielt werden können.

Diesem Hohlprofil größeren Querschnitts ist außenseitig ein Heizelement zugeordnet, wohingegen die Schraubenfeder als Kraftspeicher im Profilhohlraum verläuft.

Erfindungsgemäß ragen vom Tragorgan radiale -- symmetrisch angeordnete -- Sperrklinken als Rastorgane ab, denen als Eingriffspartner Rillen sägezahnartigen Querschnitts mit in Bewegungsrichtung querschnittlich geneigten Stirnwänden und radialen Rückwänden zugeordnet sind. Dazu hat es sich als günstig erwiesen, die Rastorgane oder Sperrklinken auf zwei gegenüberliegende Umfangsquartalen des Umfangsquerschnittes des Tragorgans oder des zentralen Ringdurchbruches der Ringescheibe/n zu begrenzen. Die Sägezahnrillen sind nach einem anderen Merkmal der Erfindung in die Innenseite des Hohlstranges oder Rohres so eingebracht, daß sie in jeweils zwei nebeneinanderliegenden Quartalen des Rohrquerschnittes gegenläufig gerichtet sind.

Jedoch gibt es auch asymmetrisch -- gegen die Bewegungsrichtung weisende -- Sperrklinken, die in einem Winkel zur Längsachse geneigt sind und denen als Eingriffspartner ebenfalls Sägezahnrillen mit in Bewegungsrichtung querschnittlich geneigten Stirnwänden und radialen Rückwänden zugeordnet sind. Hier sollen die Sperrklinken an zwei benachbarten Sechsteln des Umfangsquerschnittes des Tragorgans sowie die Sägezahnrillen an zwei benachbarten Sechsteln des Hohlstranges oder Rohres innenseitig sowie zueinander gegenläufig vorgesehen werden. Außerdem sollen zwei einander gegenüberliegende Sechstel des Umfangsquerschnittes des Tragorgans sowie des Hohlstranges oder Rohres glatt ausgebildet -- also von Klinken und Rillen frei --sein. Die beschriebenen Anordnungen der Eingriffspartner erlauben eine Umkehr der Arbeitsrichtung durch Inversion der Reibungsanisotropie, so beispielsweise durch Rotation der Sperrklinken in einen Bereich mit invertierter Reibungsanisotropie. Auch kann eine Umkehr der Arbeitsrichtung durch Umkehr des Klemm- und Rutschzyklus der Raupenfüße erreicht werden.

Von besonderer Bedeutung ist es, das aus einer Formgedächtnislegierung geformte Element als Kreissegment auszugestalten, das zwischen jeweils ihrerseits kreissegmentartig ausgebildeten Tragorganen angeordnet und vom Kraftspeicher umfangen ist, wobei diese Antriebseinheit insgesamt von teilbogenförmiger Gestalt ist.

In einer Ausgestaltung ist die Antriebseinheit dem mit wenigstens einem Bereich von Sägezahnrillen ausgestatteten Umfang einer Walze zugeordnet; zumindest eine Fläche eines Tragorgans der Antriebseinheit ist dann entsprechend dem Umfang der Walze gekrümmt und mit Sperrklinken als Eingriffspartner versehen.

In einer weiteren Ausgestaltung wird diese Antriebseinheit der Oberfläche einer Drehscheibe zugeordnet, die mit wenigstens einem Bereich radialer Sägezahnrillen versehen ist, wobei eine Flankenfläche wenigstens eines Tragorgans Sperrklinken als Eingriffspartner aufweist; die lineare Bewegung der Antriebseinheit wird durch Ansetzen derselben an einen drehbaren Torus in eine Rotation umgewandelt, so daß ein Schrittmotor entsteht.

Die beschriebene Vorrichtung soll als Linearantrieb für teleskopartig verlängerbare Elemente eingesetzt werden können, dies vor allem in teleskopartig verlängerbaren Marknägeln zur Verlängerung von Knochen; Marknägel zur Knochenverlängerung sind beispielhaft dem DE-C-39 21 972 zu entnehmen.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1 bis 4 und 6:: jeweils einen Längsschnitt durch einen erfindungsgemäßen Linearantrieb;
- Fig. 5: den Querschnitt durch Fig. 4 nach deren Linie V-V;
- Fig. 7, 9:: jeweils eine Stirnansicht eines Schrittmotors;
- Fig. 8, 10:: Seitenansichten zu Fig. 7 bzw. 9;
- Fig. 11:: eine Übersichtstabelle zu Anordnungen von Sperrklinken bzw. Sägezahnrillen für die Richtungsumkehr mit einem vergrößerten Ausschnitt aus Fig. 1 entsprechend deren Pfeil XI.

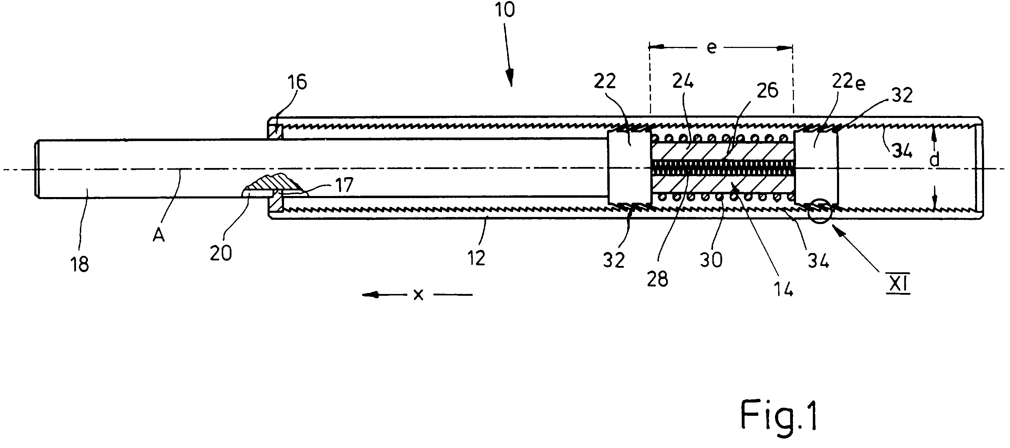

Ein Linearantrieb 10 weist gemäß Fig. 1 in einem Rohr 12 eine Antriebseinheit 14 für einen in der Rohrlängsachse A verlaufenden und in einem -- eine der Rohrmündungen verschließenden -- Gleitring 16 verschieblich gelagerten Kolben 18 auf. Dieser ist dank einer -- in eine bei 20 angedeutete achsparallele Kolbennut einragenden -- Führungsnase 17 des Gleitringes 16 gegen Verdrehen gesichert sowie mit seinem rohrwärtigen Ende an eine Diametralscheibe 22 angeschlossen.

Jene Antriebseinheit 14 enthält zwischen zwei in Axialabstand e voneinander angeordneten Diametralscheiben 22, 22ₑ einen zu Rohr 12 und Kolben 18 koaxialen Hohlzylinder 24, dessen Durchmesser geringer ist als der Innendurchmesser d des Rohres 12. Dieser Hohlzylinder 24 nimmt in seinem axialen Hohlraum 26 eine Heizspirale 28 auf, und seine Außenfläche wird von einer Schraubenfeder 30 als Energiespeicher umfangen. Letztere ist zwischen den Diametralscheiben 22, 22ₑ gespannt gelagert. Der Hohlzylinder 24 ist aus einer Formgedächtnislegierung (FGL) gefertigt, der über jene elektrische Heizspirale 28 Wärme zugeführt werden kann.

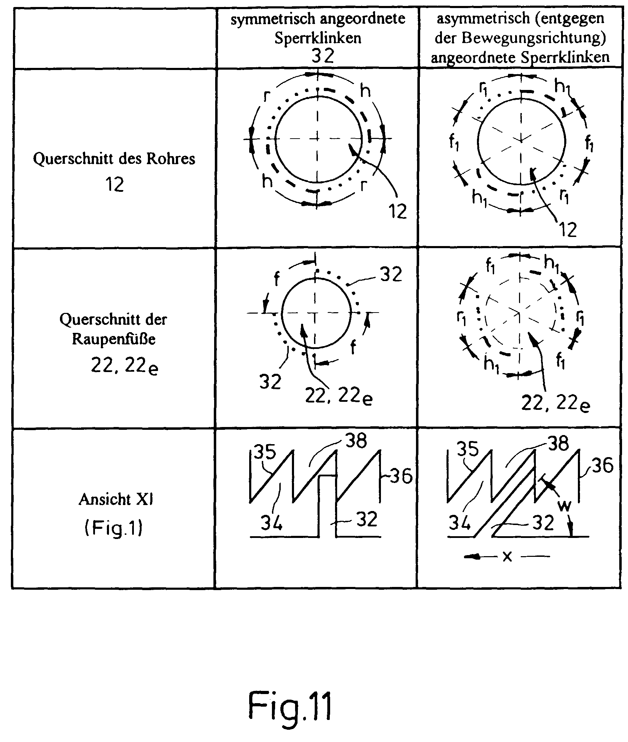

Vom Umfang der beiden Diametralscheiben 22, 22ₑ ragen streifenartige Sperrklinken 32 ab, die gemäß Fig. 11 --untere Abbildungsreihe, Felder S₃ und AS₃ -- radial oder in einem Winkel w von hier etwa 50° gegen die Bewegungsrichtung x des Kolbens 18 geneigt sein können. Diese den Diametralscheiben 22, 22ₑ die Eigenschaft von Raupenfüßen verleihenden Sperrklinken 32 kämmen mit -- in die Rohrinnenfläche eingeformten -- Rillen 34 sägezahnartigen Querschnitts. Die Sägezahnrillen 34 bieten querschnittlich in Bewegungsrichtung x jeweils eine etwa in jenem Winkel w geneigte Stirnfläche 35 sowie anderseits eine radiale Rückfläche 36; beide Flächen 35, 36 werden von einer -- zwei Sägezahnrillen 34 trennenden -- Rippenwandung 38 angeboten (Fig. 11).

Sowohl die Sperrklinken 2 als auch die Sägezahnrillen 34 sind also so ausgerichtet, daß die Sperrklinken 32 in Bewegungsrichtung x über die Rippenwandungen 38 der Sägezahnrillen 34 rutschen können, gegen die Bewegungsrichtung x aber sperren.

Wird nun beispielsweise der aus einer NiTi-Legierung geformte Hohlzylinder 24 über deren Schalttemperatur hinaus erwärmt, entsteht eine Verkürzung der Länge e des Hohlzylinders 24. Da der vordere Raupenfuß 22 sperrt, rutschen die Sperrklinken 32 des hinteren Raupenfußes 22ₑ über die Rippenwandungen 38 und bewegen sich in Richtung x. Bei diesem Vorgang erfährt auch der Kraftspeicher 30 eine Kontraktion.

Kühlt der Hohlzylinder 24 ab, ist er -- bei einer FGL mit Zweiwegeffekt -- bestrebt, wieder jene ursprüngliche Form anzunehmen; der hintere Raupenfuß 22ₑ sperrt und der vordere Raupenfuß 22 bewegt den Kolben 18 im Lasthub. Da beim Abkühlen nur etwa 30 % der Kraft gegenüber dem Erwärmen freigesetzt wird, kann ein Teil der Energie über den Kraftspeicher 30 wieder genutzt werden, so daß bei der Bewegung des vorderen Raupenfußes 22 annähernd die gleiche Energie zur Verfügung steht wie bei der Bewegung des hinteren Raupenfußes 22ₑ.

Zur Richtungsumkehr sind in Fig. 11 zwei Möglichkeiten dargestellt. Zum einen ist bei symmetrisch angeordneten Sperrklinken 32 -- in der linken Spalte der Fig. 11 -- das Rohr 12 nach Feld S₁ in vier Bereiche eingeteilt. Zwei gegenüberliegende Quartale -- beispielsweise h (hoch) -- sind mit einer Oberfläche ausgestattet, die in eine Richtung ein Sperren sowie in die andere Richtung ein Rutschen erlauben, und die beiden anderen Quartale sind mit einer gegenüber den Quartalen h richtungsinversen Oberfläche ausgestattet. Die beiden zugeordneten Raupenfüße 22, 22ₑ besitzen gemäß Feld S₂ an zwei gegenüberliegenden Quartalen symmetrische Sperrklinken 32. Die beiden Quartale f (frei) dazwischen enthalten keine Sperrklinken. Soll der Kolben 18 beispielsweise ausfahren, greifen die Sperrklinken 32 in den h-Bereich des Rohres 12 ein; der r-Bereich des Rohres 12 läuft frei. Mit einer Verdrehung des Kolbens 18 um 90° gegenüber dem Rohr 12 greifen die Sperrklinken 32 in den r-Bereich des Rohres 12 und der h-Bereich läuft frei, womit eine Richtungsumkehr erreicht wird.

Zum anderen werden bei asymmetrisch angeordneten Sperrklinken 32 -- gemäß rechter Spalte der Fig. 11 -- sowohl der Raupenfußquerschnitt -- in Feld AS₂ -- als auch der Rohrquerschnitt -- Feld AS₁ -- in h₁-, r₁- und f₁-Bereiche eingeteilt; jeweils zwei gegenüberliegende Sechstel -- also 60°des Querschnittes -- besitzen h₁-, r₁- und f₁-Bereiche. Soll der Kolben 18 beispielsweise ausfahren, greifen die beiden Sechstel der h₁-Bereiche ineinander. Die r₁-Bereiche der Raupenfüße 22, 22ₑ befinden sich im f₁-Bereich des Rohres 12 und liegen daher frei; die r₁-Bereiche des Rohres 12 und im f₁-Bereich der Raupenfüße 22, 22ₑ frei. Zur Richtungumkehr muß der Kolben 18 gegenüber dem Rohr 12 um ein Sechstel verdreht werden.

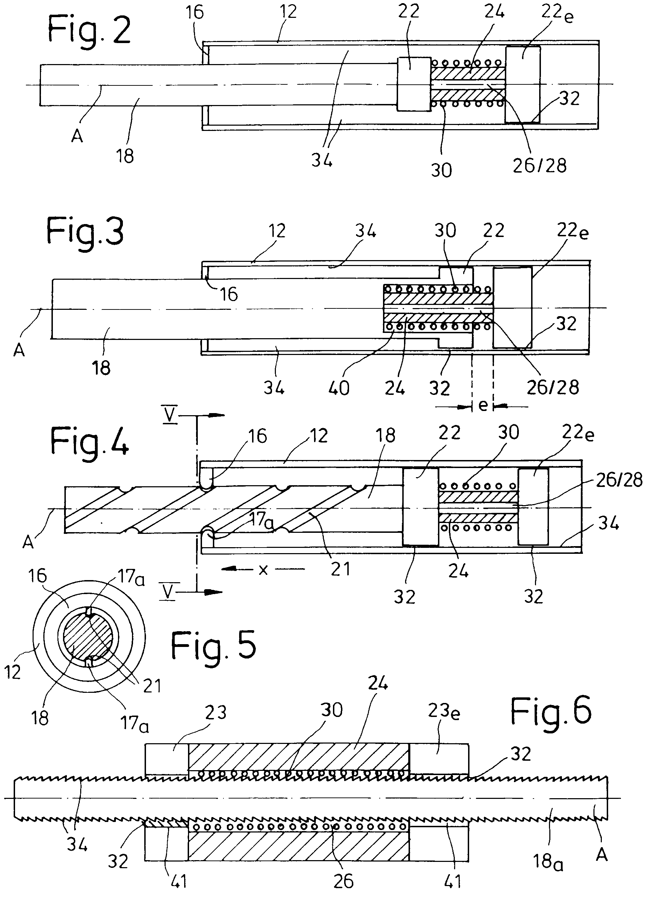

Bei der Ausführung nach Fig. 2 erfolgt die Kraftübertragung zwischen Raupenfuß 22 oder 22ₑ und dem Rohr 12 über Kraftschluß. Wird der Hohlzylinder 24 beispielsweise bei Erwärmung kürzer, so kann der vordere Raupenfuß 22 aus einem FGL-Material bestehen, das sich bei Erwärmung ausdehnt; zudem ist er von geringerem Durchmesser als der hintere Raupenfuß 22ₑ, welcher mit dem Rohr 12 kämmt. Der hintere Raupenfuß 22ₑ besteht beispielsweise aus einem Werkstoff, der bei Erwärmung schrumpft. Dabei sollten die Schalttemperaturen aller drei FGL-Materialien annähernd gleich sein. Eine weitere Möglichkeit ist, die Erwärmung sequentiell erfolgen zu lassen.

Gemäß Fig. 3 kann der vordere Raupenfuß 22 von einem Endflansch des Kolbens 18 gebildet werden. Letzterer ist in seinem hinteren Bereich mit einer axialen Sackbohrung 40 versehen, in welche ein Teil des Hohlzylinders 24 einragt, d.h. der Abstand e zwischen den Raupenfüßen 22, 22ₑ ist verhältnismäßig kurz - und unterschiedlich zur Länge des Hohlzylinders 24.

Versieht man den Kolben 18 -- wie in Fig. 4, 5 dargestellt -- mit einer Spiralnut 21, so führen in diese eingreifende Nasen 17ₐ des Gleitringes 16 den Kolben 18 in Richtung x in eine Drehung gegen den Uhrzeigersinn - bei Bewegung in Gegenrichtung entsteht dann eine gegensinnige Drehung.

Der Kolben 18ₐ der Fig. 6 ist außenseitig mit Sägezahnrillen 34 versehen; diese kämmen mit den Sperrklinken 32 von zwei Ringscheiben 23, 23ₑ, deren zylindrischer Ringdurchbruch 41 vom Kolben 18ₐ durchsetzt ist. Hier befindet sich die Antriebseinheit 14 außerhalb des Kolbens 18ₐ und umgibt diesen; in Radialabstand zu ihm liegt die Schraubenfeder 30 der Innenwandung des Hohlraumes 26 an und der diesem anbietende Hohlzylinder 24 bildet eine äußere Rohrwand. An diese sind Heizelemente von außen her anlegbar.

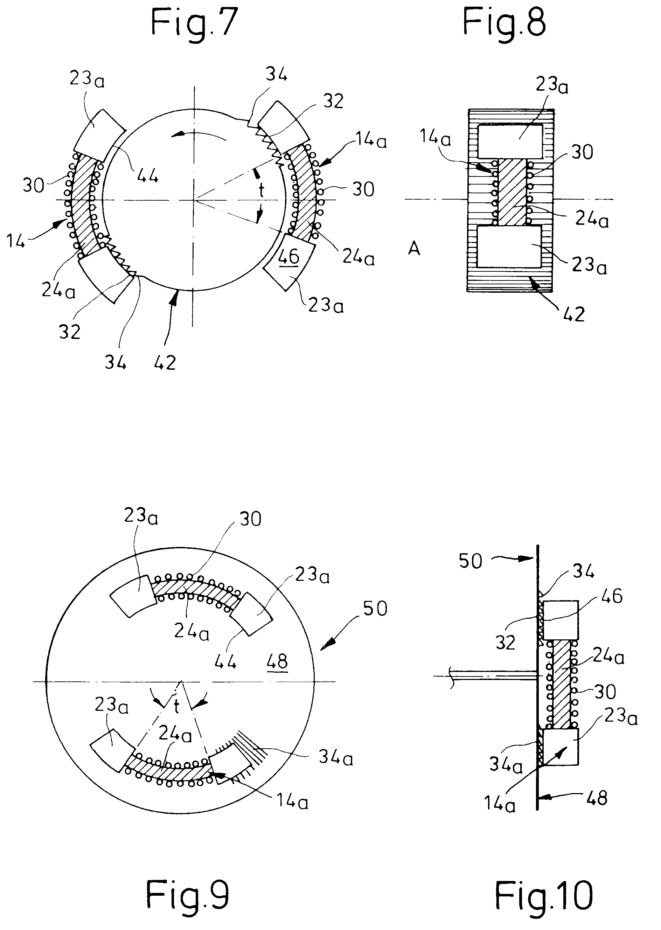

Im Ausführungsbeispiel der Fig. 7, 8 sind einer am Umfang mit Bereichen von Sägezahnrillen 34 ausgestatteten, drehbar gelagerten Walze 42 zwei -- je eine Schraubenfeder 30 tragende -- Ringsegmente 24ₐ aus FGL als Teil einer Antriebseinheit 14ₐ zugeordnet, die einen Zentralwinkel t von hier etwa 50° bestimmen. Die Stirnenden der Ringsegmente 24ₐ sind jeweils an einen Segmentkörper 23ₐ angefügt, der an jener entsprechend der Walzenoberfläche gekrümmten Innenfläche 44 die beschriebenen Sperrklinken 32 trägt.

Die Antriebseinheit 14ₐ der Fig. 9, 10 ist mit Segmentkörpern 23ₐ ausgerüstet, die jeweils an einer ihrer ebenen Flankenflächen 46 die Sperrklinken 32 tragen. Diese Antriebseinheit 14ₐ wird auf die -- Felder mit radialen Sägezahnrillen 34ₐ enthaltende -- Oberfläche 48 einer Drehscheibe 50 aufgesetzt; ihre radialen Sägezahnrillen 34ₐ können dann mit den Sperrklinken 32 der Segmentkörper 23ₐ kämmen.

## Patentansprüche

1. Antriebsvorrichtung mit einer Antriebseinheit, die ein aus einer Formgedächtnislegierung geformtes Element und einen letzterem zugeordneten, einer Arbeitsbewegung entgegenwirkenden Kraftspeicher enthält, wobei das Element unter Temperatureinwirkung gegen den Kraftspeicher längenveränderlich ausgebildet ist,
dadurch gekennzeichnet,
daß das aus der Formgedächtnislegierung geformte Element (24, 24ₐ) endwärts jeweils einem zu ihm querschnittsverschiedenen Tragorgan (22, 22ₑ; 23, 23ₑ, 23ₐ) zugeordnet und der Kraftspeicher (30) zwischen den Tragorganen spannbar ist, wobei zumindest eines der Tragorgane mit einem zu ihm relativ bewegbaren Widerlager (12, 18ₐ, 42, 50) geführt ist sowie bei Kraft- und/oder Formschluß zusammenwirkt.

2. Vorrichtung nach Anspruch 1, dadurch gekennzeichnet, daß zumindest eines der Tragorgane (22,22ₑ;23,23ₑ,23ₐ) mit Greifelementen (32) versehen ist, die mit Gegenelementen (34, 34ₐ) des zu den Tragorganen relativ bewegbaren Widerlagers (12,18ₐ,42,50) kämmend zusammenwirken.

3. Vorrichtung nach Anspruch 1 oder 2, dadurch gekennzeichnet, daß die Greifelemente (32) der Tragorgane (22, 22ₑ, 23, 23ₑ; 23ₐ) sowie die Gegenelemente (34, 34ₐ) des Widerlagers (12, 18ₐ; 42, 50) als Eingriffspartner einen Kraftschluß herstellenden Raupenmechanismus bilden zur Erzeugung eines definierten Hubes zwischen Tragorgan und Widerlager bei Durchlaufen eines Temperaturzyklus des Elementes (24, 24ₐ) aus Formgedächtnislegierung.

4. Vorrichtung nach Anspruch 1 oder 2, dadurch gekennzeichnet, daß die Greifelemente (32) der Tragorgane (22, 22ₑ; 23, 23ₑ; 23ₐ) sowie die Gegenelemente (34, 34ₐ) des Widerlagers (12, 18ₐ; 42, 50) als Eingriffspartner einen auf Reibungsanisotropie beruhenden Raupenmechanismus bilden zur Erzeugung eines definierten Hubes zwischen Tragorgan und Widerlager bei Durchlaufen eines Temperaturzyklus des Elementes (24, 24ₐ) aus Formgedächtnislegierung.

5. Vorrichtung nach Anspruch 1 oder 4, gekennzeichnet durch eine die Reibungsanisotropie vermittelnde formschlüssige Rutschverzahnung aus Fasern, ausgerichteten Lamellen oder Stufen als Eingriffspartner.

6. Vorrichtung nach Anspruch 2 oder 4, gekennzeichnet durch eine die Reibungsanisotropie vermittelnde formschlüssige Rutschverzahnung mit in rillenartige Vertiefungen (34, 34ₐ) eingreifbaren Rastorganen (32) als Eingriffspartner.

7. Vorrichtung nach einem der Ansprüche 1 bis 6, dadurch gekennzeichnet, daß ihre Antriebseinheit (14) ein Hohlprofil (24) als das aus der Formgedächtnislegierung hergestellte Element umfaßt, das in einem innenseitig einen der Eingriffspartner (34) enthaltenden Hohlstrang (12) in einer Längsachse (A) verschieblich zwischen zwei quer zur Längsachse verlaufenden scheibenartigen Tragorganen (22, 22ₑ) gelagert sowie wenigstens eines der Tragorgane mit dem anderen Eingriffspartner (32) versehen ist, wobei gegebenenfalls eines der Tragorgane (22) mit einem axial beweglich gelagerten strangartigen Kolben (18) verbunden ist.

8. Vorrichtung nach Anspruch 7 oder 8, dadurch gekennzeichnet, daß der Kolben (18) mit einer Sackbohrung (40) zur Aufnahme eines Abschnittes des Hohlprofiles (24) versehen ist, oder daß der Kolben (18) in wenigstens einem Gleitring (16) geführt ist, der bevorzugt im Hohlstrang (12) festliegt.

9. Vorrichtung nach Anspruch 7 oder 8, dadurch gekennzeichnet, daß der Kolben (18) an seinem Umfang mit zumindest einer Spiralnut (21) versehen ist, die mit wenigstens einer vom Hohlstrang (12) einwärts ragenden Nase (17ₐ) kämmt, und/oder daß der Kolben wenigstens eine achsparallele Kolbennut (20) für eine Führungsnase (17) des Hohlstranges (12) aufweist, wobei gegebenenfalls die Nase/n (17, 17ₐ) radial vom Gleitring (16) abragt/en.

10. Vorrichtung nach einem der Ansprüche 7 bis 9, dadurch gekennzeichnet, daß das Hohlprofil ein von einer Schraubenfeder als Kraftspeicher (30) umfangener Hohlzylinder (24) und/oder der Hohlstrang (12) ein Rohr sind/ist, wobei gegebenenfalls im Hohlraum (26) des Hohlzylinders (24) wenigstens ein Heizelement (28) lagert.

11. Vorrichtung nach einem der Ansprüche 1 bis 6, dadurch gekennzeichnet, daß ihre Antriebseinheit (14) ein Hohlprofil (24) als aus der Formgedächtnislegierung hergestelltes Element enthält, dessen Hohlraum (26) von einem Kolben (18ₐ) axial durchsetzt ist, wobei der Kolben einen Eingriffspartner (34) aufweist, der mit dem anderen Eingriffspartner (32) im Ringdurchbruch (41) wenigstens einer Ringscheibe (23, 23ₑ) als Tragorgan kämmt, wobei gegebenenfalls das Hohlprofil (24) ein Hohlzylinder ist.

12. Vorrichtung nach Anspruch 11, dadurch gekennzeichnet, daß sich zwischen zwei Ringscheiben (23, 23ₐ) wenigstens eine im zylindrischen Hohlraum (26) des Hohlprofils (24) vorgesehene Schraubenfeder als Kraftspeicher (30) abstützt, und/oder daß dem Hohlprofil (24) außenseitig ein Heizelement zugeordnet ist.

13. Vorrichtung nach wenigstens einem der Ansprüche 1 bis 12, dadurch gekennzeichnet, daß vom Tragorgan (22, 22ₑ; 23, 23ₑ) radiale Sperrklinken (32) als Rastorgane abragen, denen als Eingriffspartner Rillen (34, 34ₐ) sägezahnartigen Querschnitts mit in Bewegungsrichtung querschnittlich geneigten Stirnwänden (35) und radialen Rückwänden (36) zugeordnet sind.

14. Vorrichtung nach wenigstens einem der Ansprüche 6 bis 13, dadurch gekennzeichnet, daß die Rastorgane oder Sperrklinken (32) von zwei gegenüberliegenden Umfangsquartalen des Umfangsquerschnittes des Tragorgans (22, 22ₑ) oder des zentralen Ringdurchbruches (41) der Ringscheibe/n (23, 23ₑ) abragen.

15. Vorrichtung nach Anspruch 13 oder 14, dadurch gekennzeichnet, daß die Sägezahnrillen (34, 34ₐ) in die Innenseite des Hohlstranges oder Rohres (12) so eingebracht sind, daß sie in jeweils zwei nebeneinander liegenden Quartalen (h, r) des Rohrquerschnittes gegenläufig gerichtet sind.

16. Vorrichtung nach wenigstens einem der Ansprüche 1 bis 12, dadurch gekennzeichnet, daß vom Tragorgan (22, 22ₑ; 23, 23ₑ) gegen die Bewegungsrichtung (x) in einem Winkel (w) zur Längsachse (A) geneigte Sperrklinken (32) abragen, denen als Eingriffspartner Sägezahnrillen (34, 34ₐ) mit in Bewegungsrichtung querschnittlich geneigten Stirnwänden (35) und radialen Rückwänden (36) zugeordnet sind, wobei gegebenenfalls die Sperrklinken (32) an zwei benachbarten Sechsteln (h₁, r₁) des Umfangsquerschnittes des Tragorgans (22, 22ₑ) sowie die Sägezahnrillen (34, 34ₐ) an zwei benachbarten Sechsteln (h₁, r₁) des Hohlstranges oder Rohres (12) innenseitig sowie zueinander gegenläufig vorgesehen sind.

17. Vorrichtung nach Anspruch 16, dadurch gekennzeichnet, daß zwei einander gegenüberliegende Sechstel (f₁) des Umfangsquerschnittes des Tragorgans (22, 22ₑ) sowie des Hohlstranges oder Rohres (12) glatt ausgebildet sind.

18. Vorrichtung nach Anspruch 1 oder 2, dadurch gekennzeichnet, daß das aus einer Formgedächtnislegierung geformte Element (24ₐ) ein Kreissegment ist, das zwischen jeweils kreissegmentartig ausgebildeten Tragorganen (23ₐ) angeordnet und von dem Kraftspeicher (30) umfangen ist, wobei diese Antriebseinheit (14ₐ) teilbogenförmig verläuft, wobei gegebenenfalls die Antriebseinheit (14ₐ) dem mit wenigstens einem Bereich von Sägezahnrillen (34) ausgestatteten Umfang einer Walze (42) zugeordnet ist, wobei zumindest eine Fläche (44) eines Tragorgans (23ₐ) der Antriebseinheit entsprechend dem Umfang der Walze gekrümmt und mit Sperrklinken (32) als Eingriffspartner versehen ist, oder wobei gegebenenfalls die Antriebseinheit (14ₐ) der Oberfläche (48) einer Drehscheibe (50) zugeordnet und diese mit wenigstens einem Bereich radialer Sägezahnrillen (34ₐ) versehen ist, wobei eine Flankenfläche (46) wenigstens eines Tragorgans (23ₐ) Sperrklinken (32) als Eingriffspartner aufweist.

19. Verwendung eines Linearantriebs nach wenigstens einem der voraufgehenden Patentansprüche für teleskopartig verlängerbare Elemente, insbesondere für teleskopartig verlängerbare Marknägel zur Verlängerung von Knochen in situ.
